(19) Europäisches Patentamt / European Patent Office / Office européen des brevets

(11) **EP 4 480 941 A1**

(12) **EUROPEAN PATENT APPLICATION**
published in accordance with Art. 153(4) EPC

(43) Date of publication:
25.12.2024 Bulletin 2024/52

(21) Application number: 23756395.2

(22) Date of filing: 15.02.2023

(51) International Patent Classification (IPC):
*C07C 45/35* (2006.01)   *C07C 47/22* (2006.01)
*C07C 51/215* (2006.01)   *C07C 57/05* (2006.01)
*C07B 61/00* (2006.01)

(52) Cooperative Patent Classification (CPC):
(C-Sets available)
**C07C 1/24; C07B 61/00; C07C 45/35;**
**C07C 51/252;** C07C 2521/04; C07C 2521/08
(Cont.)

(86) International application number:
**PCT/JP2023/005198**

(87) International publication number:
**WO 2023/157870 (24.08.2023 Gazette 2023/34)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: 17.02.2022  JP 2022022717

(71) Applicant: **Mitsubishi Chemical Corporation**
**Tokyo 100-8251 (JP)**

(72) Inventors:
• **SUZUKI, Tatsuya**
  **Tokyo 100-8251 (JP)**
• **KATO, Yuki**
  **Tokyo 100-8251 (JP)**

(74) Representative: **Hoffmann Eitle**
**Patent- und Rechtsanwälte PartmbB**
**Arabellastraße 30**
**81925 München (DE)**

(54) **METHOD FOR PRODUCING METHACROLEIN AND/OR METHACRYLIC ACID, AND METHOD FOR PRODUCING METHACRYLIC ACID ESTER**

(57)    An object is to provide a method for producing methacrolein and/or methacrylic acid from isobutanol, in which methacrolein and/or methacrylic acid are/is obtained at high selectivity with suppressed generation of a by-product, and a method for producing methacrylic acid ester from the methacrylic acid obtained. The object is achieved by a method for producing methacrolein and/or methacrylic acid, including (i) a step of feeding an isobutanol-containing gas to a dehydration catalyst layer, to produce an isobutylene-containing gas 1 by dehydration reaction of isobutanol, and (ii) a step of feeding an isobutylene-containing gas 2 containing at least one portion of the isobutylene-containing gas 1, and an oxygen-containing gas to an oxidation catalyst layer, to produce methacrolein and/or methacrylic acid by oxidation reaction of isobutylene, in which x1/x2 is 0.4 or more when the content rate of isobutylene in the isobutylene-containing gas 1 is x1 (% by mol) and the content rate of isobutylene in the isobutylene-containing gas 2 is x2 (% by mol).

EP 4 480 941 A1

(52) Cooperative Patent Classification (CPC): (Cont.)

C-Sets
**C07C 1/24, C07C 11/09;**
**C07C 45/35, C07C 47/22;**
**C07C 51/252, C07C 57/04**

**Description**

TECHNICAL FIELD

The present invention relates to a method for producing methacrolein and/or methacrylic acid from isobutanol, and a method for producing methacrylic acid ester.

BACKGROUND ART

Methacrylic resins produced from methyl methacrylate are excellent in properties such as transparency and weather resistance, and are used in various applications. As methods for producing methacrolein and methacrylic acid serving as intermediates of methyl methacrylate, gas-phase oxidation methods with t-butanol and isobutylene as raw materials have been conventionally used.

For example, Patent Document 1 describes a method for producing methacrolein by gas-phase catalytic oxidation of t-butanol. Patent Document 2 describes a method for producing methacrolein and methacrylic acid by contacting a mixed gas containing isobutylene with a catalyst containing molybdenum and bismuth in a gas phase.

Both of these methods are methods in which petroleum-derived chemical products are conceived as starting materials. However, in recent years, there has been a concern about depletion of petroleum, and $CO_2$ generated in petroleum combustion has been considered a problem causing global warming. Isobutanol obtained by fermentation methods is then expected to serve as biomass-derived chemical products instead of those derived from petroleum.

As methods for producing methacrolein and methacrylic acid with isobutanol as a raw material, for example, Patent Document 3 describes a method with a fixed bed reactor in which the former stage is filled with a dehydration catalyst and the latter stage is filled with an oxidation catalyst. Patent Document 4 describes a method for producing methacrolein and methacrylic acid, including producing t-butanol from isobutanol and dehydrating and oxidizing the resulting t-butanol.

RELATED ART DOCUMENT

PATENT DOCUMENT

Patent Document 1: JP-A S48-32814
Patent Document 2: JP-A S50-13308
Patent Document 3: JP-A 2013-121946
Patent Document 4: WO 2013/069630

SUMMARY OF THE INVENTION

PROBLEMS TO BE SOLVED BY THE INVENTION

However, no sufficient selectivity of methacrolein and methacrylic acid is obtained in the above methods for producing methacrolein and methacrylic acid with isobutanol as a raw material, and a further enhancement in selectivity is desired.

An object of the present invention is to provide a method for producing methacrolein and/or methacrylic acid from isobutanol, in which methacrolein and/or methacrylic acid are/is obtained at high selectivity with suppressed generation of a by-product. An object of the present invention is to provide a method for producing methacrylic acid ester from the methacrylic acid obtained.

MEANS FOR SOLVING THE PROBLEMS

The present invention has the following aspects.

[1] : A method for producing methacrolein and/or methacrylic acid from isobutanol, including

(i) a step of feeding an isobutanol-containing gas to a dehydration catalyst layer, to produce an isobutylene-containing gas 1 by dehydration reaction of isobutanol, and
(ii) a step of feeding an isobutylene-containing gas 2 containing at least one portion of the isobutylene-containing gas 1, and an oxygen-containing gas to an oxidation catalyst layer, to produce methacrolein and/or methacrylic acid by oxidation reaction of isobutylene, wherein

x1/x2 is 0.4 or more when the content rate of isobutylene in the isobutylene-containing gas 1 is x1 (% by mol) and the content rate of isobutylene in the isobutylene-containing gas 2 is x2 (% by mol) .

[2] : The method for producing methacrolein and/or methacrylic acid according to [1], wherein the x1/x2 is 0.6 or more.

[3] : The method for producing methacrolein and/or methacrylic acid according to [1] or [2], wherein the content rate of oxygen in the isobutanol-containing gas in the step (i) is 8% by mol or less.

[4] : The method for producing methacrolein and/or methacrylic acid according to any of [1] to [3], wherein the content rate of oxygen in the isobutanol-containing gas in the step (i) is 5% by mol or less.

[5] : The method for producing methacrolein and/or methacrylic acid according to any of [1] to [4], wherein the content rate of isobutanol in the isobutanol-containing gas in the step (i) is 5% by mol or more.

[6]: The method for producing methacrolein and/or methacrylic acid according to any of [1] to [5], wherein a mixed gas obtained by mixing water with the isobutylene-containing gas 2 and gasifying the resulting mixture is fed to the oxidation catalyst layer in the step (ii).

[7]: The method for producing methacrolein and/or methacrylic acid according to any of [1] to [6], wherein dehydration reaction of isobutanol is performed at 260 to 380°C in the step (i).

[8]: The method for producing methacrolein and/or methacrylic acid according to any of [1] to [7], wherein the conversion rate of isobutanol in the step (i) is 95%.

[9]: The method for producing methacrolein and/or methacrylic acid according to any of [1] to [8], wherein oxidation reaction of isobutylene is performed at 200 to 400°C in the step (ii).

[10]: A method for producing methacrylic acid, including oxidizing methacrolein produced by the method according to any [1] to [9], to produce methacrylic acid.

[11]: A method for producing methacrylic acid ester, including esterifying methacrylic acid produced by the method according to any [1] to [10], to produce methacrylic acid ester.

EFFECTS OF THE INVENTION

According to the present invention, methacrolein and/or methacrylic acid can be produced at high selectivity with suppressed generation of a by-product in a method for producing methacrolein and/or methacrylic acid from isobutanol.

MODE FOR CARRYING OUT THE INVENTION

Hereinafter, embodiments according to the present invention are described, but the present invention is not limited to the following.

Herein, a numerical value range expressed with "to" means a range including numerical values described before and after "to" respectively as the lower limit value and the upper limit value, and "A to B" means A or more and B or less.

[Method for producing methacrolein and/or methacrylic acid]

A production method according to an embodiment of the present invention is a method for producing methacrolein and/or methacrylic acid from isobutanol, and has the following steps (i) and (ii).

(i) A step of feeding an isobutanol-containing gas to a dehydration catalyst layer, to produce an isobutylene-containing gas 1 by dehydration reaction of isobutanol.

(ii) A step of feeding an isobutylene-containing gas 2 containing at least one portion of the isobutylene-containing gas 1, and an oxygen-containing gas to an oxidation catalyst layer, to produce methacrolein and/or methacrylic acid by oxidation reaction of isobutylene.

In a production method according to an embodiment of the present invention, x1/x2 is 0.4 or more when the content rate of isobutylene in the isobutylene-containing gas 1 is x1 (% by mol) and the content rate of isobutylene in the isobutylene-containing gas 2 is x2 (% by mol).

Such a method can be used to produce methacrolein and/or methacrylic acid at high selectivity with suppressed generation of a by-product.

Hereinafter, each step is described in detail.

<Step (i)>

In step (i), an isobutanol-containing gas is fed to a dehydration catalyst layer, to produce an isobutylene-containing gas 1 by dehydration reaction of isobutanol.

(Dehydration catalyst layer)

The dehydration catalyst layer can be formed by filling a reactor for dehydration reaction of isobutanol (hereinafter, also referred to as "reactor 1"), with a dehydration catalyst. The dehydration catalyst here used can be an acid catalyst, and specific examples thereof include alumina, silica alumina, silica aluminophosphate, aluminophosphate, zeolite, solid phosphoric acid, zirconia, and composite oxides constituted from tungsten, niobium, and the like. The dehydration catalyst preferably contains alumina from the viewpoint of selectivity of isobutylene in step (i). The content rate of alumina based on the total mass of the dehydration catalyst is preferably 90% by mass or more, more preferably 95% by mass or more, further preferably 97% by mass or more, particularly preferably 98% by mass or more, especially preferably 99% by mass or more, most preferably 99.5% by mass or more. The dehydration catalyst may be used singly or in combination of two or more kinds thereof. The content rate of alumina in the dehydration catalyst is a value determined by ICP emission spectrometric analysis. ICP emission spectrometric analysis can be performed with, for example, Optima 8300 ICP-OES Spectrometer (product name, manufactured by Perkin Elmer).

When the dehydration catalyst contains alumina, the crystal form of alumina is not particularly limited, and various types of alumina, such as $\alpha$-alumina, $\beta$-alumina, $\gamma$-alumina, $\sigma$-alumina, $\theta$-alumina, $\delta$-alumina, and alumina hydrate, can be used. In particular, $\gamma$-alumina is preferably contained from the viewpoint of selectiveness of isobutylene. Such alumina may be used singly in one crystal form or in combination of two or more crystal forms. When two or more crystal forms are used in combination, different crystal forms may be mixed or a mixed phase crystal state may be taken.

It is preferable that 90% by mass or more of the dehydration catalyst have a particle size in the range of 700 to 10000 um. The dehydration catalyst, which has a particle size of 700 um or more, is used to result in a reduction in pressure loss of the dehydration catalyst layer. Thus, the facility cost and the energy cost can be decreased and a reduction in reactivity due to an increase in reaction pressure can be suppressed. The dehydration catalyst, which has a particle size of 10000 um or less, is used to result in an increase in effectiveness factor of the catalyst and an enhancement in activity per mass of the catalyst. The lower limit of the particle size is more preferably 800 um or more, further preferably 1000 um or more. The upper limit of the particle size is more preferably 9500 um or less, further preferably 9000 um or less. The dehydration catalyst may be, if necessary, molded, and when the shape of the catalyst is any other shape than a spherical shape, the length in a direction at which the maximum length is observed is defined as the particle size.

The BET specific surface area of the dehydration catalyst is preferably 30 to 1000 $m^2/g$. When the BET specific surface area is 30 $m^2/g$ or more, the catalyst activity is enhanced, and when the BET specific surface area is 1000 $m^2/g$ or less, the catalyst stability is enhanced. The lower limit of the BET specific surface area is more preferably 40 $m^2/g$ or more, further preferably 50 $m^2/g$ or more, particularly preferably 60 $m^2/g$ or more, especially preferably 70 $m^2/g$ or less, most preferably 80 $m^2/g$ or more. The upper limit of the BET specific surface area is more preferably 800 $m^2/g$ or less, further preferably 600 $m^2/g$ or less, particularly preferably 400 $m^2/g$ or less, especially preferably 350 $m^2/g$ or less, most preferably 300 $m^2/g$ or less. The BET specific surface area of the dehydration catalyst is a value calculated from an $N_2$ adsorption/desorption isotherm, and can be measured with, for example, TriStar 3000 (product name, manufactured by Shimadzu Corporation).

The dehydration catalyst layer may be an undiluted layer of only the dehydration catalyst, or may be a diluted layer further including an inert carrier. The dehydration catalyst layer may be a single layer, or a mixed layer including a plurality of layers.

(Reactor 1)

The reactor 1 is not particularly restricted as long as it is an apparatus in which gas-phase isobutanol can be allowed to pass through a reactor, thereby performing dehydration reaction, and isobutylene as a product of the reaction can be drawn. The reactor here used can be known one, and examples thereof include fixed bed and fluid bed reactors.

In the case of a fixed bed or fluid bed reactor, the location of the dehydration catalyst layer in the reactor, the proportion of the dehydration catalyst layer in the reactor, and the like are not particularly limited, and any form commonly used can be applied.

(Isobutanol-containing gas)

The isobutanol-containing gas can be prepared by, for example, gasifying a raw material containing isobutanol, by a gasifier. The gasifier is not particularly limited, and examples thereof include jacket type, natural-circulation horizontal tube type, natural-circulation immersion tube type, natural-circulation vertical short tube type, vertical long tube rising film film type, horizontal tube falling film type, forced-circulation horizontal tube type, forced-circulation vertical tube type, and coil type gasifiers. The gasification temperature is preferably 120 to 400°C, and the pressure is preferably 50 to 1000 kPa as the absolute pressure.

The isobutanol used in the isobutanol-containing gas is preferably biomass-derived isobutanol in terms of environmental protection. The biomass-derived isobutanol here refers to isobutanol purified from an organic compound obtained through a fermentation process with fermentable sugar of biomass, or isobutanol obtained by a process including any one or more of catalytic chemical conversion and thermochemical conversion of biomass. Biomass is roughly classified to those derived from resource crops and those derived from waste. Examples of biomass derived from resource crops include edible crops, wood, and grass and flowers, and unutilized portions of these crops can also be used. Examples of biomass derived from waste include food waste, sludge such as sewage, livestock manure, and waste paper.

The content rate of oxygen in the isobutanol-containing gas is preferably 8% by mol or less. Thus, burning reaction can be suppressed during dehydration reaction of isobutanol and isobutylene can be selectively produced, thereby resulting in an enhancement in selectivity of methacrolein and/or methacrylic acid in step (ii) described below. The upper limit of the content rate of oxygen is preferably 5% by mol or less, more preferably 2.5% by mol or less, further preferably 1% by mol or less, particularly preferably 0.5% by mol or less, especially preferably 0.1% by mol or less, most preferably 0.01% by mol or less.

The lower limit of the content rate of isobutanol in the isobutanol-containing gas is preferably 5% by mol or more. Thus, the selectivity of isobutylene can be enhanced and the reactor 1 can be reduced in size, and therefore the facility cost and the energy cost can be reduced. The lower limit of the content rate of isobutanol is more preferably 10% by mol or more, further preferably 15% by mol or more, particularly preferably 20% by mol or more, especially preferably 30% by mol or more, most preferably 40% by mol or more. The upper limit of the content rate of isobutanol is not particularly limited, and can be, for example, 99.99% by mol or less.

The content rate of isobutanol in the isobutanol-containing gas can be adjusted with water vapor. The content rate of water vapor in the isobutanol-containing gas is preferably 0.01 to 80% by mol. Thus, the selectivity of isobutylene in step (i) is enhanced and the selectivity of methacrolein and methacrylic acid in step (ii) described below is enhanced. The upper limit of the content rate of water vapor is more preferably 80% by mol or less, further preferably 70% by mol or less, particularly preferably 60% by mol or less, especially preferably 50% by mol, most preferably 40% by mol or less.

The isobutanol-containing gas may contain an inert gas having no influence on dehydration reaction. Examples of the inert gas include nitrogen, helium, neon, krypton, xenon, radon, argon, methane, ethane, propane, butane, isobutane, carbon monoxide, carbon dioxide, nitrogen monoxide, nitrogen dioxide, nitrous oxide, dinitrogen trioxide, dinitrogen tetraoxide and dinitrogen pentoxide.

(Dehydration reaction of isobutanol)

The isobutanol-containing gas is preferably fed so as to be contacted with the dehydration catalyst at a linear velocity of 1.2 cm/sec or more in dehydration reaction of isobutanol. Thus, the dehydration catalyst can be inhibited from being reduced in activity and the selectivity of isobutylene is enhanced. The lower limit of the linear velocity is more preferably 1.4 cm/sec or more, further preferably 1.6 cm/sec or more, particularly preferably 1.8 cm/sec or more, especially preferably 2 cm/sec or more, most preferably 2.2 cm/sec or more. The upper limit of the linear velocity is not particularly limited, and can be, for example, 1000 cm/sec or less.

The linear velocity of the isobutanol-containing gas is defined as follows.

Linear velocity of isobutanol-containing gas (cm/sec) = Flow rate of isobutanol-containing gas (L/hour) $\times$ 1000/3600/ Cross-sectional area of reaction tube (cm$^2$)

Flow rate of isobutanol-containing gas (L/hour) = Flow rate of isobutanol-containing gas measured in normal state (NL/hour) $\times$ 101.3 (kPa)/Reaction pressure (kPa) $\times$ Reaction temperature (K)/273

The reaction pressure in the dehydration reaction is not particularly limited, and the absolute pressure thereof can be, for example, 50 to 5000 kPa or less. The reaction pressure is a value measured with a pressure sensor disposed at a location where the influence of the pressure loss on the pressure at an inlet of the reactor 1 is ignorable.

The reaction temperature in the dehydration reaction is preferably 260 to 380°C from the viewpoint of catalyst activity and selectivity of isobutylene. The lower limit of the reaction temperature is more preferably 270°C or more, further preferably 280°C or more, particularly preferably 290°C or more, most preferably 300°C or more. The reaction temperature is the lowest temperature among temperatures of the dehydration catalyst layer in a static state of the dehydration reaction. When the dehydration catalyst layer has a temperature distribution, it is preferable to increase the number of positions for temperature measurement and/or perform continuous temperature measurement in a flow direction of the isobutanol-containing gas. The method for controlling the reaction temperature is not particularly limited, and a known method can be adopted.

The conversion rate of isobutanol in the dehydration reaction is preferably 95% or more. Thus, the selectivity of methacrolein and/or methacrylic acid in step (ii) described below is enhanced. The lower limit of the conversion rate of isobutanol is more preferably 97.5% or more, further preferably 99.5% or more. The conversion rate of isobutanol can be adjusted by, for example, changing the reaction temperature in the dehydration reaction, and/or the ratio of the weight of the dehydration catalyst and the feeding rate of the isobutanol-containing gas.

(Isobutylene-containing gas 1)

The isobutylene-containing gas 1 produced in step (i) includes isobutylene, a linear alkene compound, isobutane, diisobutyl ether, isobutylaldehyde and water vapor as products. The linear alkene compound means propylene, 1-butene, cis-2-butene, and trans-2-butene.

When the content rate of isobutylene in the isobutylene-containing gas 1 is x1 (% by mol), the lower limit of x1 is preferably 1 or more, more preferably 5 or more, further preferably 7 or more, particularly preferably 10 or more. The upper limit is 50 or less, preferably 49 or less, more preferably 48 or less, further preferably 47 or less, particularly preferably 46 or less. When the content rate of the linear alkene compound in the isobutylene-containing gas 1 is y1 (% by mol), the x1/y1 is preferably 1 to 199. Here, x1 and y1 are each a value determined by gas chromatography of a gas collected through an outlet of the reactor 1 after a lapse of 5 minutes from stabilization of the reaction temperature and the reaction pressure in the reactor 1 respectively within variations of $\pm$ 0.5°C and $\pm$ 0.5 kPa. x1 and y1 can be adjusted by, for example, changing the reaction temperature in the dehydration reaction, and/or the content rate of isobutanol in the isobutanol-containing gas.

<Step (ii)>

In step (ii), an isobutylene-containing gas 2 containing at least one portion of the isobutylene-containing gas 1 produced in step (i), and an oxygen-containing gas are fed to an oxidation catalyst layer, to produce a methacrolein and/or methacrylic acid by oxidation reaction of isobutylene.

Step (i) and step (ii) are preferably connected by a pipe kept warm and/or warmed to 100°C or more. Thus, the variation in pressure due to aggregation of water vapor in the isobutylene-containing gas 1 can be suppressed. The lower limit of the temperature of the pipe is more preferably 120°C or more, further preferably 140°C or more, particularly preferably 160°C or more, most preferably 180°C or more. The upper limit is not particularly restricted, and can be, for example, 400°C or less.

(Oxidation catalyst layer)

The oxidation catalyst layer can be formed by filling a reactor for oxidation reaction of isobutylene (hereinafter, also referred to as "reactor 2"), with an oxidation catalyst. The oxidation catalyst here used can be a known catalyst, a catalyst containing at least molybdenum and bismuth is preferably used, and a catalyst having a composition represented by the following formula (1) is more preferably used.

$$Mo_{12}Bi_{a1}Fe_{a2}M_{a3}X_{a4}Y_{a5}Z_{a6}O_{a7} \qquad (1)$$

In the formula (1), Mo, Bi, Fe and O respectively represent molybdenum, bismuth, iron, and oxygen. M represents at least one element selected from the group consisting of cobalt and nickel. X represents at least one element selected from the group consisting of chromium, lead, manganese, calcium, magnesium, niobium, silver, barium, tin, tantalum, and zinc. Y represents at least one element selected from the group consisting of phosphorus, boron, sulfur, selenium, tellurium, cerium, tungsten, antimony, and titanium. Z represents at least one element selected from the group consisting of lithium, sodium, potassium, rubidium, cesium and thallium, a1 to a7 each represent the atomic ratio of each element, a1 = 0.01 to 3, a2 = 0.01 to 5, a3 = 1 to 12, a4 = 0 to 8, a5 = 0 to 5, and a6 = 0.001 to 2 are satisfied, and a7 represents the atomic ratio of oxygen necessary for satisfying the atomic valence of each element described above.

The atomic ratio of each element is a value determined by analyzing each component in the catalyst dissolved in hydrochloric acid with ICP emission spectroscopy. ICP emission spectrometric analysis can be performed with, for example, Optima 8300 ICP-OES Spectrometer (product name, manufactured by Perkin Elmer).

The oxidation catalyst may be an undiluted layer of only the oxidation catalyst, or may be a diluted layer further including an inert carrier. The dehydration catalyst layer may be a single layer, or a mixed layer including a plurality of layers.

(Reactor 2)

The reactor 2 may be an apparatus in which gas-phase isobutylene can be allowed to pass through a reactor, thereby performing oxidation reaction, and methacrolein and methacrylic acid as reaction products can be drawn. The reactor here used can be known one, and examples thereof include fixed bed and fluid bed reactors.

In the case of a fixed bed or fluid bed reactor, the location of the oxidation catalyst layer in the reactor, the proportion of the oxidation catalyst layer in the reactor, and the like are not particularly limited, and any form commonly used can be applied.

(Isobutylene-containing gas 2)

The isobutylene-containing gas 2 contains at least one portion of the isobutylene-containing gas 1 produced in step (i). When the content rate of isobutylene in the isobutylene-containing gas 2 is $x2$ (% by mol), the $x1/x2$ is 0.4 or more. Thus, the selectivity of methacrolein and methacrylic acid obtained is enhanced. It is noted that the isobutylene-containing gas 2 does not contain components newly mixed, such as an oxygen-containing gas, water, and other component.

The $x1/x2$ can be adjusted by separating or removing any component other than isobutylene, from the isobutylene-containing gas 1. The reason why the selectivity of methacrolein and methacrylic acid obtained is enhanced in the case of an $x1/x2$ of 0.4 or more, although is not clear, is considered because a trace amount of impurities contained in the isobutylene-containing gas 1, in particular, a component such as diisobutyl ether contributes to an enhancement in selectivity of methacrolein and/or methacrylic acid in oxidation reaction of isobutylene. Therefore, the selectivity of methacrolein and/or methacrylic acid is considered to be enhanced by feeding the isobutylene-containing gas 2, which shows an $x1/x2$ of 0.4 or more, namely, which includes a certain or more amount of impurities in the isobutylene-containing gas 1, in step (ii).

The $x1/x2$ is preferably 0.6 or more, more preferably 0.8 or more, further preferably 0.9 or more, particularly preferably 0.95 or more, most preferably 1. Here, $x2$ is a value measured by gas chromatography after separation or removal of any component from the isobutylene-containing gas 1. In gas chromatography, for example, GC-8A-FID (manufactured by Shimadzu Corporation, column: Unicarbon A-400 4m manufactured by GL Sciences Inc., inner diameter 3 mm, carrier gas: helium) can be used. When the whole amount of the isobutylene-containing gas 1 is used as the isobutylene-containing gas 2 without separation or removal of any component from the isobutylene-containing gas 1, $x2 = x1$ may be satisfied.

The isobutylene-containing gas 2 preferably includes 60% by mol or more, more preferably 70% by mol or more, further preferably 80% by mol or more, particularly preferably 90% by mol or more, especially preferably 95% by mol or more, most preferably 99% by mol or more of the isobutylene-containing gas 1.

(Oxygen-containing gas)

The selectivity of methacrolein and methacrylic acid is enhanced by feeding the oxygen-containing gas to the oxidation catalyst layer in step (ii). The oxygen-containing gas here used is preferably air, or can be, if necessary, air enriched with pure oxygen.

The oxygen-containing gas is preferably fed so that 0.1 to 5 mol of oxygen is fed based on 1 mol of isobutylene in the isobutylene-containing gas 2, and the lower limit is more preferably 0.5 mol or more and the upper limit is more preferably 3 mol or less.

The oxygen-containing gas may be mixed with the isobutylene-containing gas 2 and then fed to the oxidation catalyst layer, or may be fed to the oxidation catalyst layer separately from the isobutylene-containing gas 2.

(Water and other component)

A mixed gas obtained by mixing water with the isobutylene-containing gas 2 and gasifying the resulting mixture is preferably fed to the oxidation catalyst layer in step (ii). After mixing of water, for example, a jacket type, natural-circulation horizontal tube type, natural-circulation immersion tube type, natural-circulation vertical short tube type, vertical long tube rising film type, horizontal tube falling film type, forced-circulation horizontal tube type, forced-circulation vertical tube type, or coil type gasifier can be used for gasification. The gasification temperature is preferably 120 to 400°C, and the pressure is preferably 50 to 1000 kPa as the absolute pressure.

The content rate of water in the mixed gas is preferably 1 to 45% by mol.

A mixed gas obtained by further mixing an inert gas as other component with the isobutylene-containing gas 2 may be fed to the oxidation catalyst layer. Examples of the inert gas include nitrogen, helium, neon, krypton, xenon, radon, argon, methane, ethane, propane, butane, isobutane, carbon monoxide, carbon dioxide, nitrogen monoxide, nitrogen dioxide, nitrous oxide, dinitrogen trioxide, dinitrogen tetraoxide and dinitrogen pentaoxide.

(Oxidation reaction of isobutylene)

The lower limit of the contact time of the isobutylene-containing gas 2 with the oxidation catalyst in oxidation reaction of isobutylene is preferably 0.5 seconds or more, more preferably 1 second or more. The upper limit is preferably 10 seconds or less, more preferably 6 seconds or less.

The reaction pressure in the oxidation reaction is not particularly limited, and the absolute pressure thereof can be, for example, 50 to 5000 kPa or less. The reaction pressure is a value measured with a pressure sensor disposed at a location where the influence of the pressure loss on the pressure at an inlet of the reactor 2 is ignorable.

The reaction temperature in the oxidation reaction is preferably 200 to 400°C from the viewpoint of selectivity of methacrolein and methacrylic acid. The lower limit of the reaction temperature is more preferably 210°C or more, further preferably 220°C or more, particularly preferably 230°C or more, especially preferably 240°C or more, most preferably 250°C or more, from the viewpoint of selectivity of methacrolein and/or methacrylic acid. The reaction temperature is the highest temperature among temperatures of the oxidation catalyst layer in a static state of the oxidation reaction. When the oxidation catalyst layer has a temperature distribution, it is preferable to increase the number of positions for temperature measurement and/or perform continuous temperature measurement in a flow direction of the isobutylene-containing gas 2. The method for controlling the reaction temperature is not particularly limited, and a known method can be adopted.

[Method for producing methacrylic acid]

A method for producing methacrylic acid according to an embodiment of the present invention includes oxidizing methacrolein produced by a method according to an embodiment of the present invention, to produce methacrylic acid. Thus, methacrylic acid can be produced from isobutanol at high selectivity.

A method for producing methacrylic acid according to an embodiment of the present invention can be specifically carried out by contacting a catalyst for methacrylic acid production and a raw material gas containing methacrolein in a reactor. The reactor for methacrylic acid production, here used, can be one commonly used in gas-phase oxidation, and is preferably a tubular reactor provided with a reaction tube having a catalyst layer. A multi-tubular reactor provided with a plurality of the reaction tubes is preferably used in terms of industry.

(Catalyst for methacrylic acid production)

The catalyst for methacrylic acid production, here used, can be a known catalyst, a catalyst containing at least molybdenum and phosphorus is preferably used, and a catalyst having a composition represented by the following formula (2) is more preferably used. The atomic ratio of each element is a value determined by analyzing the component of the catalyst dissolved in ammonia water with ICP emission spectroscopy.

$$P_{a8}Mo_{12}V_{a9}CU_{a10}A_{a11}E_{a12}G_{a13}O_{a14} \qquad (2)$$

In the formula (2), P, Mo, V, Cu and O respectively represent phosphorus, molybdenum, vanadium, copper, and oxygen. A represents at least one element selected from the group consisting of antimony, bismuth, arsenicum, germanium, zirconium, tellurium, silver, selenium, silicon, tungsten, and boron. E represents at least one element selected from the group consisting of potassium, rubidium, cesium, thallium, magnesium and barium. G represents at least one element selected from the group consisting of iron, zinc, chromium, calcium, strontium, tantalum, cobalt, nickel, manganese, titanium, tin, lead, niobium, indium, sulfur, palladium, gallium, cerium and lanthanum. a8 to a14 each represent the atomic ratio of each element, $a8 = 0.5$ to 3, $a9 = 0.01$ to 3, $a10 = 0.01$ to 2, and $a11 = 0$ to 3, preferably 0.01 to 3, $a12 = 0.01$ to 3 and $a13 = 0$ to 4 are satisfied, and a14 represents the atomic ratio of oxygen necessary for satisfying the atomic valence of each element.

The catalyst layer may be an undiluted layer of only the catalyst for methacrylic acid production, or may be a diluted layer further including an inert carrier. The dehydration catalyst layer may be a single layer, or a mixed layer including a plurality of layers.

(Raw material gas)

The concentration of methacrolein in the raw material gas is preferably 1 to 20% by volume, the lower limit is more preferably 3% by volume or more, and the upper limit is more preferably 10% by volume or less.

The oxygen source of the raw material gas, here used, is air in economic terms, or may be, if necessary, air enriched with pure oxygen. The concentration of oxygen in the raw material gas is preferably 0.5 to 4 mol based on 1 mol of methacrolein, and the lower limit is more preferably 1 mol or more and the upper limit is more preferably 3 mol or less.

The raw material gas may contain a small amount of impurities such as lower saturated aldehyde, and such an amount is preferably smaller, if possible. The raw material gas may be diluted with an inert gas such as nitrogen or a carbon dioxide gas, water vapor, or the like.

(Oxidation reaction of methacrolein)

The reaction pressure in oxidation reaction of methacrolein is usually about atmospheric pressure to several atm. The reaction temperature is preferably 230 to 450°C or more, and the lower limit is more preferably 250°C or more and the upper limit is more preferably 400°C or less.

[Method for producing methacrylic acid ester]

A method for producing methacrylic acid ester according to an embodiment of the present invention includes esterifying methacrylic acid produced by a method according to an embodiment of the present invention, to produce methacrylic acid ester. Thus, methacrylic acid ester can be produced from isobutanol at high selectivity.

A method for producing methacrylic acid ester according to an embodiment of the present invention can be specifically carried out by feeding a raw material fluid including methacrylic acid and alcohol, into a reactor, and subjecting the fluid to esterification reaction.

The recovery and purification step of the methacrylic acid ester produced can be simplified by setting the composition of the raw material fluid from the viewpoint of chemical equilibrium so that the concentration of any one of methacrylic acid and alcohol is higher and the conversion rate of such a raw material lower in concentration is higher. The alcohol is not particularly limited, and saturated and unsaturated alcohols each having 1 to 10 carbon atoms in its molecule can be used.

An esterification catalyst here used is preferably an acid catalyst, or can be, for example, sulfuric acid or an ion exchange resin. The ion exchange resin is preferably a strongly acidic cation exchange resin, and specific examples thereof include DIAION (registered trademark), PK216, and RCP12H (manufactured by Mitsubishi Chemical Corporation), Lewatit (registered trademark), K2431 (manufactured by Bayer AG), and Amberlyst (registered trademark) 15WET (manufactured by ROHM AND HAAS JAPAN K.K.). These may be used singly or in combination of two or more kinds thereof.

The flow direction of the reaction fluid in the reactor for methacrylic acid ester production can be appropriately selected, and the flow direction of the reaction fluid is preferably perpendicularly upward when an ion exchange resin to be largely swollen is used as the esterification catalyst. The flow direction of the reaction fluid is preferably perpendicularly downward when the reaction fluid forms an heterogeneous phase.

The reaction temperature in the esterification reaction is preferably 40 to 130°C. When the reaction temperature is 40°C or more, the reaction rate is increased and the esterification reaction can be efficiently carried out. When the reaction temperature is 130°C or less, the esterification catalyst is inhibited from being degraded, and continuous running can be made for a long time.

EXAMPLES

Hereinafter, the present invention is specifically described with reference to Examples, but the present invention is not limited by the following description.

(Compositional ratio of oxidation catalyst)

The atomic ratio of each element was determined by analyzing each component in the catalyst dissolved in hydrochloric acid with ICP emission spectroscopy. Optima 8300 ICP-OES Spectrometer (product name, manufactured by Perkin Elmer) was used in such ICP emission spectrometric analysis.

(Evaluation of dehydration reaction)

Evaluation of dehydration reaction was initiated after a lapse of 5 minutes from stabilization of the reaction temperature and the reaction pressure in the reactor 1 respectively within variations of $\pm$ 0.5°C and $\pm$ 0.5 kPa.

A gas collected through an outlet of the reactor 1 was absorbed by water, and a gas component not dissolved in water was collected by a syringe and analyzed with gas chromatography. The gas chromatography used in the analysis is shown below.

Amounts of isobutylene, propylene, isobutane, 1-butene, cis-2-butene, and trans-2-butene produced: GC-8A-FID (manufactured by Shimadzu Corporation, column: Unicarbon A-400 4m manufactured by GL Sciences Inc., inner diameter 3 mm, carrier gas: helium)
Amount of carbon dioxide produced: GC-2014-TCD (manufactured by Shimadzu Corporation, column: PorapakQ 2m manufactured by GL Sciences Inc., inner diameter 3 mm, carrier gas: helium)
Amount of carbon monoxide produced: GC-2014-TCD (manufactured by Shimadzu Corporation, column: Molecular sieve 13X2m manufactured by GL Sciences Inc., inner diameter 3 mm, carrier gas: helium)

The gas collected through an outlet of the reactor 1 was absorbed by ice-cooled acetonitrile (manufactured by FUJIFILM Wako Pure Chemical Corporation, special grade reagent) for 1 hour and recovered in a measuring flask, and acetonitrile was added for adjustment to 250 mL. Next, the resultant was split in a 20-ml measuring flask, and 0.3 g of 2-propanol (manufactured by FUJIFILM Wako Pure Chemical Corporation, special grade reagent) was added as an internal standard. The resulting absorption liquid was subjected to gas chromatography (GC-2010 (manufactured by Shimadzu Corporation, column: DB-FFAP manufactured by J&W, 30 m × 0.32 mm, film thickness 0.25 um, carrier gas: hydrogen)), to analyze the amount of the remaining unreacted isobutanol.

(Evaluation of oxidation reaction)

Evaluation of oxidation reaction was initiated after a lapse of 60 minutes from stabilization of the reaction temperature and the reaction pressure in the reactor 2 respectively within variations of ± 0.5°C and ± 0.5 kPa.
A gas collected through an outlet of the reactor 2 was absorbed by water, and a gas component not dissolved in water was collected by a syringe and analyzed with gas chromatography. The gas chromatography used in the analysis is shown below.

Amounts of propylene, isobutane, 1-butene, cis-2-butene, trans-2-butene, and 1,3-butadiene produced, and amount of remaining unreacted isobutylene: GC-8A-FID (manufactured by Shimadzu Corporation, column: Unicarbon A-400 4m manufactured by GL Sciences Inc., inner diameter 3 mm, carrier gas: helium)
Amount of carbon dioxide produced: GC-2014-TCD (manufactured by Shimadzu Corporation, column: PorapakQ 2m manufactured by GL Sciences Inc., inner diameter 3 mm, carrier gas: helium)
Amount of carbon monoxide produced: GC-2014-TCD (manufactured by Shimadzu Corporation, column: Molecular sieve 13X2m manufactured by GL Sciences Inc., inner diameter 3 mm, carrier gas: helium)

The gas collected through an outlet of the reactor 2 was absorbed by ice-cooled acetonitrile (manufactured by FUJIFILM Wako Pure Chemical Corporation, special grade reagent) for 1 hour and recovered in a measuring flask, and acetonitrile was added for adjustment to 250 mL. Next, the resultant was split in a 20-ml measuring flask, and 0.3 g of 2-propanol (manufactured by FUJIFILM Wako Pure Chemical Corporation, special grade reagent) was added as an internal standard. The resulting absorption liquid was subjected to gas chromatography (GC-2010 (manufactured by Shimadzu Corporation, column: DB-FFAP manufactured by J&W, 30 m × 0.32 mm, film thickness 0.25 um, carrier gas: hydrogen)), to analyze the amounts of acetone, methacrolein, acetic acid and methacrylic acid produced, and the amount of the remaining unreacted isobutanol.

(Conversion rate of raw material, selectivity of product, and x1, x2 and y1)

The conversion rate of each raw material, the selectivity of each product, and x1, x2 and y1 were calculated from the above analysis results of gas chromatography, according to the following expressions.

<Conversion rate of each raw material and selectivity of each product in Examples 1 to 5 and Comparative Examples 1 to 2>

· Step (i)

```
Conversion rate of isobutanol (%) = ((a - b)/a) × 100

  Selectivity of isobutylene (%) = (c/(a - b)) × 100

Selectivity of linear alkene compound = (d/(a - b)) × 100
```

$$\text{Selectivity of isobutane} = (e/(a - b)) \times 100$$

Selectivity of diisobutyl ether and isobutylaldehyde = $(f/(a - b)) \times 100$

· Step (ii) (based on the amount of isobutylene produced in step (i))

$$\text{Conversion rate of isobutylene (\%)} = ((h - i)/h) \times 100$$

$$\text{Selectivity of methacrolein (\%)} = (k/(h - i)) \times 100$$

$$\text{Selectivity of methacrylic acid (\%)} = (l/(h - i)) \times 100$$

$$\text{Selectivity of acetone (\%)} = 3/4 \times (m/(h - i)) \times 100$$

$$\text{Selectivity of acetic acid (\%)} = 1/2 \times (n/(h - i)) \times 100$$

$$\text{Selectivity of isobutane (\%)} = (q/(h - i)) \times 100$$

$$\text{Selectivity of COx (\%)} = (r/(h - i)) \times 100$$

· Step (ii) (based on the amount of isobutanol fed to step (i))

$$\text{Conversion rate of isobutanol (\%)} = ((a - j)/a) \times 100$$

$$\text{Selectivity of isobutylene (\%)} = (i/(a - j)) \times 100$$

$$\text{Selectivity of methacrolein (\%)} = (k/(a - j) \times 100$$

$$\text{Selectivity of methacrylic acid (\%)} = (l/(a - j)) \times 100$$

$$\text{Selectivity of acetone (\%)} = 3/4 \times (m/(a - j)) \times 100$$

$$\text{Selectivity of acetic acid (\%)} = 1/2 \times (n/(a - j)) \times 100$$

Selectivity of linear alkene compound (%) = $(o/(a - j)) \times 100$

$$\text{Selectivity of 1,3-butadiene (\%)} = (p/(a - j)) \times 100$$

$$\text{Selectivity of isobutane (\%)} = (q/(a - j)) \times 100$$

$$\text{Selectivity of COx (\%)} = (r/(a - j)) \times 100$$

<Conversion rate of each raw material and selectivity of each product in Comparative Example 3>

Conversion rate of isobutanol (%) = $((a'' - j'')/a'') \times 100$

$$\text{Selectivity of isobutylene (\%)} = (i''/(a'' - j'')) \times 100$$

$$\text{Selectivity of methacrolein (\%)} = (k''/(a'' - j'')) \times 100$$

Selectivity of methacrylic acid (%) = (l"/(a" - j")) × 100

$$\text{Selectivity of acetone (\%)} = 3/4 \times (m''/(a'' - j'')) \times 100$$

Selectivity of acetic acid (%) = 1/2 × (n"/(a" - j")) × 100 Selectivity of linear alkene compound (%) = (o"/(a" - j")) × 100

$$\text{Selectivity of 1,3-butadiene (\%)} = (p''/(a'' - j'')) \times 100$$

$$\text{Selectivity of isobutane (\%)} = (q''/(a'' - j'')) \times 100$$

$$\text{Selectivity of COx (\%)} = (r''/(a'' - j'')) \times 100$$

<x1, x2 and y1>

$$x1 = c/(b + c + d + e + f + g)$$

$$y1 = d/(b + c + d + e + f + g)$$

$$x2 = c'/(b' + c' + d' + e' + f' + g')$$

Herein, the meaning of each symbol in the above expressions is as follows.

a: Number of moles of isobutanol fed to reactor 1
b: Number of moles of isobutanol in gas collected through the outlet of reactor 1
c: Number of moles of isobutylene in gas collected through the outlet of reactor 1
d: Number of moles of linear alkene compound in gas collected through the outlet of reactor 1
e: Number of moles of isobutane in gas collected through the outlet of reactor 1
f: Number of moles of diisobutyl ether and isobutylaldehyde in gas collected through the outlet of reactor 1
g: Number of moles of nitrogen, oxygen, water, carbon monoxide and carbon dioxide in gas collected through the outlet of reactor 1
b': Number of moles of isobutanol in isobutylene-containing gas 2
c': Number of moles of isobutylene in isobutylene-containing gas 2
d': Number of moles of linear alkene compound in isobutylene-containing gas 2
e': Number of moles of isobutane in isobutylene-containing gas 2
f': Number of moles of diisobutyl ether and isobutylaldehyde in isobutylene-containing gas 2
g': Number of moles of nitrogen, oxygen, water, carbon monoxide and carbon dioxide in isobutylene-containing gas 2
h: Number of moles of isobutylene fed to reactor 2
i: Number of moles of isobutylene in gas collected through the outlet of reactor 2
j: Number of moles of isobutanol in gas collected through the outlet of reactor 2
k: Number of moles of methacrolein in gas collected through the outlet of reactor 2
l: Number of moles of methacrylic acid in gas collected through the outlet of reactor 2
m: Number of moles of acetone in gas collected through the outlet of reactor 2

n: Number of moles of acetic acid in gas collected through the outlet of reactor 2

o: Number of moles of linear alkene compound in gas collected through the outlet of reactor 2

p: Number of moles of 1,3-butadiene in gas collected through the outlet of reactor 2

q: Number of moles of isobutane in gas collected through the outlet of reactor 2

r: Number of moles of carbon monoxide and carbon dioxide in gas collected through the outlet of reactor 2

a": Number of moles of isobutanol fed to reactor 3

i": Number of moles of isobutylene in gas collected through the outlet of reactor 3

j'': Number of moles of isobutanol in gas collected through the outlet of reactor 3

k'': Number of moles of methacrolein in gas collected through the outlet of reactor 3

l": Number of moles of methacrylic acid in gas collected through the outlet of reactor 3

m": Number of moles of acetone in gas collected through the outlet of reactor 3

n'': Number of moles of acetic acid in gas collected through the outlet of reactor 3

o'': Number of moles of linear alkene compound in gas collected through the outlet of reactor 3

p'': Number of moles of 1,3-butadiene in gas collected through the outlet of reactor 3

q'': Number of moles of isobutane in gas collected through the outlet of reactor 3

r": Number of carbon monoxide and carbon dioxide in gas collected through the outlet of reactor 3

[Catalyst Production Example 1]

An alumina molded article shaped into a cylindrical pellet having a diameter of 3.0 mm (main component of crystal phase: $\gamma$-alumina phase, BET specific surface area: 105 $m^2$/g, content rate of $Na_2O$: less than 0.05% by mass, content rate of $SiO_2$: 0.16% by mass) was ground and granulated so that the proportion of particles having a particle size of 800 to 1190 um was 90% by mass or more, and thus a dehydration catalyst 1 was obtained.

[Catalyst Production Example 2]

A liquid A was prepared by mixing 100 parts of ammonium molybdate tetrahydrate, 6 parts of cesium nitrate, 8 parts of bismuth trioxide and 5 parts of antimony trioxide with 400 parts of pure water at 60°C. A liquid B was prepared by mixing 38 parts of iron nitrate nonahydrate, 89 parts of cobalt nitrate hexahydrate and 14 parts of nickel nitrate hexahydrate with 200 parts of pure water. Next, the liquid A and the liquid B were mixed and heated, and stirred at 90°C for 1 hour, and thus a slurry-like liquid C was obtained.

Most of the water in the liquid C obtained was gasified, and thus a cake-like product was obtained. The cake-like product was heat-treated under an air atmosphere at 120°C for 16 hours, further heat-treated under an air atmosphere at 300°C for 1 hour, and then pulverized. The pulverized product was pressure-molded and then pulverized, the resulting ground particles were classified, and those passing through a sieve having an aperture of 2.36 mm and not passing through a sieve having an aperture of 0.71 mm were recovered. The resulting ground particles after classification were heat-treated under an air atmosphere at 500°C for 6 hours, and thus an oxidation catalyst 1 was obtained. The composition of the oxidation catalyst 1, except for oxygen, was $Mo_{12}Bi_{0.7}Fe_{2.0}Ni_{1.0}Co_{6.5}Sb_{0.7}Cs_{0.6}$.

[Example 1]

<Step (i)>

A vertical tubular reaction tube having an inner diameter of 0.50 cm and a length of 40 cm was used as the reactor 1, and was filled with 1.0000 g of the dehydration catalyst 1, to form a dehydration catalyst layer.

An isobutanol-containing gas was prepared by feeding isobutanol (manufactured by NACALAI TESQUE, INC., content rate of water: 411 ppm) and nitrogen as an inert gas to a gasifier, and gasifying them at 200°C. Here, isobutanol was fed at 3.4 mL/hour by use of a syringe pump and nitrogen was fed at 3300 mL (standard state)/hour by use of a mass flow meter. The composition of the isobutanol-containing gas obtained is shown in Table 1.

Next, the isobutanol-containing gas was fed to the reactor 1 so as to be contacted with the dehydration catalyst 1 at a linear velocity of 11.94 cm/sec, and dehydration reaction was performed at a reaction temperature of 341°C, thereby producing the isobutylene-containing gas 1. The evaluation results of dehydration reaction and x1 are shown in Table 1.

<Step (ii)>

A vertical tubular reaction tube having an inner diameter of 0.75 cm and a length of 40 cm was used as the reactor 2, and filled with 6.0024 g of the oxidation catalyst 1, to form an oxidation catalyst layer.

The total amount of the isobutylene-containing gas 1 obtained in step (i) was used as the isobutylene-containing gas 2, and fed to a gasifier through a pipe kept warm at 200°C. Here, separation or removal of any component from the isobutylene-containing gas 1 was not performed. x2 and x1/x2 are shown in Table 2.

Next, water, air and nitrogen were fed respectively at 0.66 mL/hour, 9840 mL (standard state)/hour and 960 mL (standard state)/hour, to a gasifier by use of a syringe pump and a mass flow meter, and mixed with the isobutylene-containing gas 2 and gasified, to prepare a mixed gas. The composition of the mixed gas obtained is shown in Table 2.

Next, the mixed gas at 16369 mL (standard state)/hour was fed to the reactor 2, and oxidation reaction was performed at a reaction temperature of 341°C. The evaluation results of oxidation reaction are shown in Table 2.

[Example 2]

<Step (i)>

The same reactor 1 as in Example 1 was used and filled with 1.0000 g of the dehydration catalyst 1, to form a dehydration catalyst layer.

An isobutanol-containing gas was prepared by the same method as in Example 1 except that nitrogen was fed at 840 ml (standard state)/hour to a gasifier. The composition of the isobutanol-containing gas obtained is shown in Table 1.

Next, the isobutanol-containing gas was fed to the reactor 1 so as to be contacted with the dehydration catalyst 1 at a linear velocity of 4.80 cm/sec, and dehydration reaction was performed at a reaction temperature of 340°C, thereby producing the isobutylene-containing gas 1. The evaluation results of dehydration reaction and x1 are shown in Table 1.

<Step (ii)>

The same reactor 2 as in Example 1 was used and filled with 6.0028 g of the oxidation catalyst 1, to form an oxidation catalyst layer.

The total amount of the isobutylene-containing gas 1 obtained in step (i) was used as the isobutylene-containing gas 2, and fed to a gasifier through a pipe kept warm at 200°C. Here, separation or removal of any component from the isobutylene-containing gas 1 was not performed. x2 and x1/x2 are shown in Table 2.

Next, a mixed gas was prepared by the same method as in Example 1 except that nitrogen was fed at 3420 mL (standard state)/hour to a gasifier. The composition of the mixed gas obtained is shown in Table 2.

Next, the mixed gas was fed at 15671 mL (standard state)/hour to the reactor 2, and oxidation reaction was performed at a reaction temperature of 340°C. The evaluation results of oxidation reaction are shown in Table 2.

[Example 3]

<Step (i)>

The same reactor 1 as in Example 1 was used and filled with 1.0000 g of the dehydration catalyst 1, to form a dehydration catalyst layer.

An isobutanol-containing gas was prepared by the same method as in Example 1 except that no nitrogen was fed to a gasifier. The composition of the isobutanol-containing gas obtained is shown in Table 1.

Next, the isobutanol-containing gas was fed to the reactor 1 so as to be contacted with the dehydration catalyst 1 at a linear velocity of 2.28 cm/sec, and dehydration reaction was performed at a reaction temperature of 341°C, thereby producing the isobutylene-containing gas 1. The evaluation results of dehydration reaction and x1 are shown in Table 1.

<Step (ii)>

The same reactor 2 as in Example 1 was used and filled with 6.0028 g of the oxidation catalyst 1, to form an oxidation catalyst layer.

The total amount of the isobutylene-containing gas 1 obtained in step (i) was used as the isobutylene-containing gas 2, and fed to a gasifier through a pipe kept warm at 200°C. Here, separation or removal of any component from the isobutylene-containing gas 1 was not performed. x2 and x1/x2 are shown in Table 2.

Next, a mixed gas was prepared by the same method as in Example 1 except that nitrogen was fed at 4260 mL (standard state)/hour to a gasifier. The composition of the mixed gas obtained is shown in Table 2.

Next, the mixed gas was fed at 16480 mL (standard state)/hour to the reactor 2, and oxidation reaction was performed at a reaction temperature of 340°C. The evaluation results of oxidation reaction are shown in Table 2.

[Example 4]

<Step (i)>

The same reactor 1 as in Example 1 was used and filled with 1.0000 g of the dehydration catalyst 1, to form a dehydration catalyst layer.

An isobutanol-containing gas was prepared by the same method as in Example 1 except that nitrogen was fed at 4680 mL (standard state)/hour to a gasifier. The composition of the isobutanol-containing gas obtained is shown in Table 1.

Next, the isobutanol-containing gas was fed to the reactor 1 so as to be contacted with the dehydration catalyst 1 at a linear velocity of 16.09 cm/sec, and dehydration reaction was performed at a reaction temperature of 340°C, thereby producing the isobutylene-containing gas 1. The evaluation results of dehydration reaction and x1 are shown in Table 1.

<Step (ii)>

The same reactor 2 as in Example 1 was used and filled with 6.0028 g of the oxidation catalyst 1, to form an oxidation catalyst layer.

An isobutylene-containing gas 2 was prepared by bubbling the isobutylene-containing gas 1 obtained in step (i) into water at 20°C and removing organic components such as unreacted isobutanol, water and diisobutyl ether, and fed to a gasifier. x2 and x1/x2 are shown in Table 2.

Next, a mixed gas was prepared by the same method as in Example 1 except that water, air, and nitrogen were fed respectively at 1.3 mL/hour, 10020 mL (standard state)/hour, and 0 mL (standard state)/hour to a gasifier. The composition of the mixed gas obtained is shown in Table 2.

Next, the mixed gas was fed at 17071 mL (standard state)/hour to the reactor 2, and oxidation reaction was performed at a reaction temperature of 340°C. The evaluation results of oxidation reaction are shown in Table 2.

[Example 5]

<Step (i)>

The same reactor 1 as in Example 1 was used and filled with 2.0000 g of the dehydration catalyst 1, to form a dehydration catalyst layer.

An isobutanol-containing gas was prepared by the same method as in Example 1 except that water, air, and nitrogen were fed respectively at 2 mL/hour, 3120 mL (standard state)/hour, and 2160 mL/hour to a gasifier. The composition of the isobutanol-containing gas obtained is shown in Table 1.

Next, the isobutanol-containing gas was fed to the reactor 1 so as to be contacted with the dehydration catalyst 1 at a linear velocity of 25.13 cm/sec, and dehydration reaction was performed at a reaction temperature of 340°C, thereby producing the isobutylene-containing gas 1. The evaluation results of dehydration reaction and x1 are shown in Table 1.

<Step (ii)>

The same reactor 2 as in Example 1 was used and filled with 6.0068 g of the oxidation catalyst 1, to form an oxidation catalyst layer.

An isobutylene-containing gas 2 was prepared with the isobutylene-containing gas 1 obtained in step (i), by the same method as in Example 4, and fed to a gasifier. x2 and x1/x2 are shown in Table 2.

Next, a mixed gas was prepared by the same method as in Example 1 except that water, air, and nitrogen were fed respectively at 1.4 mL/hour, 6720 mL (standard state)/hour, and 2100 mL (standard state)/hour to a gasifier. The composition of the mixed gas obtained is shown in Table 2.

Next, the mixed gas was fed at 16462 mL (standard state)/hour to the reactor 2, and oxidation reaction was performed at a reaction temperature of 340°C. The evaluation results of oxidation reaction are shown in Table 2.

[Comparative Example 1]

<Step (i)>

The same reactor 1 as in Example 1 was used and filled with 1.0091 g of the dehydration catalyst 1, to form a dehydration catalyst layer.

An isobutanol-containing gas was prepared by feeding isobutanol (manufactured by NACALAI TESQUE, INC., content rate of water: 411 ppm), nitrogen and air to a gasifier, and gasifying them at 200°C. Here, isobutanol was fed at 1.5 mL/hour by use of a syringe pump, and nitrogen and air were fed respectively at 1860 ml (standard state)/hour and 4320 ml (standard state)/hour by use of a mass flow meter. The composition of the isobutanol-containing gas obtained is shown in Table 1.

Next, the isobutanol-containing gas was fed to the reactor 1 so as to be contacted with the dehydration catalyst 1 at a linear velocity of 18.15 cm/sec, and dehydration reaction was performed at a reaction temperature of 340°C, thereby producing the isobutylene-containing gas 1. The evaluation results of dehydration reaction and x1 are shown in Table 1.

<Step (ii)>

The same reactor 2 as in Example 1 was used and filled with 6.0028 g of the oxidation catalyst 1, to form an oxidation catalyst layer.

The total amount of the isobutylene-containing gas 1 obtained in step (i) was used as the isobutylene-containing gas 2, and fed to a gasifier through a pipe kept warm at 200°C. Here, separation or removal of any component from the isobutylene-containing gas 1 was not performed. x2 and x1/x2 are shown in Table 2.

Next, water was fed at 0.29 mL/hour to a gasifier by use of a syringe pump, and mixed with the isobutylene-containing gas 2 and gasified, to prepare a mixed gas. In other words, a mixed gas was prepared with no oxygen-containing gas. The composition of the mixed gas obtained is shown in Table 2.

Next, the mixed gas was fed at 6923 mL (standard state)/hour to the reactor 2, and oxidation reaction was performed at a reaction temperature of 338°C. The evaluation results of oxidation reaction are shown in Table 2.

[Comparative Example 2]

<Step (i)>

The same reactor 1 as in Example 1 was used and filled with 1.0023 g of the dehydration catalyst 1, to form a dehydration catalyst layer.

An isobutanol-containing gas was prepared by the same method as in Example 1 except that water, air and nitrogen were fed respectively at 4.2 mL/hour, 2520 mL (standard state)/hour, and 0 mL/hour to a gasifier. The composition of the isobutanol-containing gas obtained is shown in Table 1.

Next, the isobutanol-containing gas was fed to the reactor 1 so as to be contacted with the dehydration catalyst 1 at a linear velocity of 24.40 cm/sec, and dehydration reaction was performed at a reaction temperature of 340°C, thereby producing the isobutylene-containing gas 1. The evaluation results of dehydration reaction and x1 are shown in Table 1.

<Step (ii)>

The same reactor 2 as in Example 1 was used, and filled with 6.0019 g of the oxidation catalyst 1, to form an oxidation catalyst layer.

An isobutylene-containing gas 2 was prepared with the isobutylene-containing gas 1 obtained in step (i), by the same method as in Example 4, and fed to a gasifier. x2 and x1/x2 are shown in Table 2.

Next, a mixed gas was prepared by the same method as in Example 1 except that water, air, and nitrogen were fed respectively at 1.4 mL/hour, 7320 mL (standard state)/hour, and 4260 mL (standard state)/hour to a gasifier. The composition of the mixed gas obtained is shown in Table 2.

Next, the mixed gas was fed at 16289 mL (standard state)/hour to the reactor 2, and oxidation reaction was performed at a reaction temperature of 340°C. The evaluation results of oxidation reaction are shown in Table 2.

[Comparative Example 3]

A vertical tubular reaction tube having an inner diameter of 1.00 cm and a length of 40 cm was used as the reactor 1, and filled with 0.3912 g of the dehydration catalyst 1 and 2.0045 g of the oxidation catalyst 1. In other words, a reactor having both the dehydration catalyst layer and the oxidation catalyst layer was used.

An isobutanol-containing gas was prepared by feeing isobutanol (manufactured by NACALAI TESQUE, INC., content rate of water: 411 ppm), water, air and nitrogen to a gasifier and gasifying them at 200°C. Here, isobutanol and water were fed respectively at 1.7 mL/hour and 0.32 mL/hour by use of a syringe pump. In addition, air and nitrogen were fed respectively at 4800 ml (standard state)/hour and 2100 ml (standard state)/hour by use of a mass flow meter. The composition of the isobutanol-containing gas obtained is shown in Table 3.

Next, the isobutanol-containing gas was fed to the reactor through the dehydration catalyst layer so as to be contacted with the dehydration catalyst 1 at a linear velocity of 5.85 cm/sec, and dehydration reaction and oxidation reaction was performed at a reaction temperature of 340°C. In other words, methacrolein and/or methacrylic acid were/was produced by a method including simultaneously performing dehydration reaction of isobutanol and oxidation reaction of isobutylene without steps (i) and (ii). The evaluation results are shown in Table 3. Here, reaction evaluation was made under the same conditions as those of the oxidation reaction.

[Table 1]

Table 1

| | | Example 1 | Example 2 | Example 3 | Comparative Example 1 | Example 4 | Example 5 | Comparative Example 2 |
|---|---|---|---|---|---|---|---|---|
| Amount of dehydration catalyst [g] | | 1.0000 | 1.0000 | 1.0000 | 1.0091 | 1.0000 | 2.0000 | 1.0023 |
| Composition of isobuta-nol-contain-ing gas [% by mol] | Isobutanol | 19.81 | 49.22 | 99.82 | 5.50 | 14.96 | 9.83 | 9.58 |
| | Water vapor | 0.04 | 0.09 | 0.18 | 0.01 | 0.03 | 26.89 | 61.12 |
| | Oxygen | 0.00 | 0.00 | 0.00 | 13.84 | 0.00 | 7.88 | 6.20 |
| | Nitrogen | 80.15 | 50.69 | 0.00 | 80.65 | 85.01 | 55.40 | 23.10 |
| Reaction temperature [° C] | | 341 | 340 | 341 | 340.00 | 340 | 340 | 340 |
| Conversion rate of isobutanol [%] | | 100.00 | 100.00 | 100.00 | 100.00 | 100.00 | 93.81 | 62.11 |
| Selectivity [%] | Isobutylene | 92.94 | 93.60 | 93.80 | 57.55 | 92.50 | 61.48 | 78.21 |
| | Linear alkene compound | 6.97 | 6.32 | 3.12 | 4.18 | 7.06 | 4.58 | 6.99 |
| | Isobutane | 0.03 | 0.04 | 0.00 | 0.00 | 0.02 | 0.00 | 0.01 |
| | Diisobutyl ether and isobutylaldehyde | 0.06 | 0.05 | 0.08 | 26.82 | 0.42 | 7.73 | 11.72 |
| $x_1$ [mol%] | | 14.7 | 31.5 | 46.5 | 1.2 | 11.2 | 5.1 | 4.5 |
| $y_1$ [ml%] | | 1.1 | 2.1 | 3.0 | 0.1 | 0.9 | 0.3 | 0.4 |

EP 4 480 941 A1

[Table 2]

[Table 2]

Table 2

|  |  |  | Example 1 | Example 2 | Example 3 | Comparative Example 1 | Example 4 | Example 5 | Comparative Example 2 |
|---|---|---|---|---|---|---|---|---|---|
| x2 [mol%] |  |  | 14.7 | 31.5 | 46.5 | 1.2 | 12.9 | 8.4 | 140 |
| x1/x2 |  |  | 1.00 | 1.00 | 1.00 | 1.00 | 0.87 | 0.61 | 0.32 |
| Amount of oxidation catalyst [g] |  |  | 6.0024 | 6.0028 | 6.0028 | 6.0028 | 6.0028 | 6.0068 | 6.0019 |
| Composition of mixed gas [% by mol] | Isobutylene |  | 4.47 | 4.57 | 4.58 | 3.73 | 6.52 | 4.58 | 3.04 |
|  | Water vapor |  | 989 | 9.89 | 9.89 | 10.21 | 15.02 | 16.11 | 10.69 |
|  | Oxygen |  | 12.61 | 12.58 | 12.58 | 12.45 | 19.56 | 19.14 | 12.70 |
|  | Nitrogen |  | 73.03 | 72.96 | 72.95 | 73.61 | 58.90 | 60.17 | 73.57 |
| Reaction temperature [° C] |  |  | 341 | 340 | 340 | 338 | 340 | 340 | 340 |
| Based on amount of isobutylene produced in step (i) | Conversion rate of isobutylene [%] |  | 93.97 | 91.53 | 91.40 | 96.93 | 93.88 | 98.30 | 79.70 |
|  | Selectivity [%] | Methacrolein | 90.50 | 91.83 | 92.06 | 86.86 | 87.60 | 78.98 | 71.70 |
|  |  | Methacrylic acid | 2.86 | 2.05 | 2.00 | 2.31 | 3.13 | 2.64 | 1.73 |
|  |  | Acetone | 0.28 | 0.40 | 0.44 | 0.75 | 0.80 | 3.41 | 7.60 |
|  |  | Acetic acid | 1.16 | 0.13 | 0.06 | 1.45 | 2.20 | 7.55 | 8.00 |
|  |  | Isobutane | 0.07 | 0.05 | 0.07 | 0.08 | 0.03 | 0.00 | 0.00 |
|  |  | Cox | 5.13 | 5.54 | 5.37 | 8.55 | 6.24 | 7.42 | 10.97 |

(continued)

| | | | Example 1 | Example 2 | Example 3 | Comparative Example 1 | Example 4 | Example 5 | Comparative Example 2 |
|---|---|---|---|---|---|---|---|---|---|
| | Conversion rate of isobutanol [%] | | 100.00 | 100.00 | 100.00 | 100.00 | 100.00 | 100.00 | 100.00 |
| Based on amount of isobutanol fed to step (i) | Selectivity [%] | Isobutylene | 6.34 | 9.09 | 9.34 | 3.11 | 6.99 | 3.05 | 19.42 |
| | | Methacrolein | 77.95 | 76.95 | 77.05 | 64.07 | 75.06 | 73.25 | 54.46 |
| | | Methacrylic acid | 2.46 | 1.72 | 1.68 | 1.70 | 2.69 | 2.45 | 1.32 |
| | | Acetone | 0.23 | 0.34 | 0.37 | 0.56 | 0.69 | 3.16 | 5.77 |
| | | Acetic acid | 0.99 | 0.11 | 0.05 | 1.07 | 1.89 | 7.00 | 6.07 |
| | | Linear alkene compound | 5.11 | 4.72 | 4.68 | 11.70 | 4.35 | 1.54 | 4.65 |
| | | 1,3-Butadiene | 2.55 | 2.47 | 2.37 | 11.51 | 3.05 | 3.25 | 1.63 |
| | | Isobutane | 0.05 | 0.05 | 0.06 | 0.06 | 0.03 | 0.00 | 0.00 |
| | | Cox | 4.32 | 4.55 | 4.40 | 6.22 | 5.25 | 6.30 | 6.68 |

[Table 3]

Table 3

|  |  |  | Comparative Example 3 |
|---|---|---|---|
| Amount of dehydration catalyst [g] | | | 0.3912 |
| Amount of oxidation catalyst [g] | | | 2.0045 |
| Composition of isobutanol-containing gas [% by mol] | Isobutanol | | 5.29 |
| | Water vapor | | 8.31 |
| | Oxygen | | 13.02 |
| | Nitrogen | | 73.38 |
| Reaction temperature [° C] | | | 340 |
| Based on amount of isobutanol fed | Conversion rate of isobutanol [%] | | 100.00 |
| | Selectivity [%] | Methacrolein | 43.75 |
| | | Methacrylic acid | 0.17 |
| | | Acetone | 7.32 |
| | | Acetic acid | 3.72 |
| | | Isobutane | 0.01 |
| | | Isobutylene | 22.21 |
| | | Linear alkene | 5.02 |
| | | 1,3-Butadiene | 0.87 |
| | | Cox | 16.93 |

As shown in Table 2, Examples 1 to 3, in which similar values of x1/x2 were exhibited and the oxygen-containing gas was fed to the oxidation catalyst layer in step (ii), resulted in higher total selectivity of methacrolein and methacrylic acid than that in Comparative Example 1 in which no oxygen-containing gas was fed in step (ii). Examples 1 to 5, in which the x1/x2 was equal to or more than 0.4 prescribed, resulted in higher total selectivity of methacrolein and methacrylic acid than that in Comparative Example 2 in which the x1/x2 was less than 0.4.

As shown in Table 3, Comparative Example 3, in which no steps (i) and (ii) were included and dehydration reaction and oxidation reaction were performed with a reactor having both the dehydration catalyst layer and oxidation catalyst layer, resulted in lower total selectivity of methacrolein and methacrylic acid than those in Examples 1 to 5 shown in Table 2, each including steps (i) and (ii).

Here, methacrolein obtained in the present Examples can be oxidized, thereby obtaining methacrylic acid, and such methacrylic acid can be esterified, thereby obtaining methacrylic acid ester.

INDUSTRIAL APPLICABILITY

According to the present invention, there can be provided a method for obtaining methacrolein and/or methacrylic acid at high selectivity with suppressed generation of a by-product.

**Claims**

1. A method for producing methacrolein and/or methacrylic acid from isobutanol, comprising

(i) a step of feeding an isobutanol-containing gas to a dehydration catalyst layer, to produce an isobutylene-containing gas 1 by dehydration reaction of isobutanol, and
(ii) a step of feeding an isobutylene-containing gas 2 containing at least one portion of the isobutylene-containing gas 1, and an oxygen-containing gas to an oxidation catalyst layer, to produce methacrolein and/or methacrylic acid, wherein

x1/x2 is 0.4 or more when the content rate of isobutylene in the isobutylene-containing gas 1 is x1 (% by mol) and the content rate of isobutylene in the isobutylene-containing gas 2 is x2 (% by mol) .

2. The method for producing methacrolein and/or methacrylic acid according to claim 1, wherein the x1/x2 is 0.6 or more.

3. The method for producing methacrolein and/or methacrylic acid according to claim 1 or 2, wherein the content rate of oxygen in the isobutanol-containing gas in the step (i) is 8% by mol or less.

4. The method for producing methacrolein and/or methacrylic acid according to any one of claims 1 to 3, wherein the content rate of oxygen in the isobutanol-containing gas in the step (i) is 5% by mol or less.

5. The method for producing methacrolein and/or methacrylic acid according to any one of claims 1 to 4, wherein the content rate of isobutanol in the isobutanol-containing gas in the step (i) is 5% by mol or more.

6. The method for producing methacrolein and/or methacrylic acid according to any one of claims 1 to 5, wherein a mixed gas obtained by mixing water with the isobutylene-containing gas 2 and gasifying the resulting mixture is fed to the oxidation catalyst layer in the step (ii).

7. The method for producing methacrolein and/or methacrylic acid according to any one of claims 1 to 6, wherein dehydration reaction of isobutanol is performed at 260 to 380°C in the step (i).

8. The method for producing methacrolein and/or methacrylic acid according to any one of claims 1 to 7, wherein the conversion rate of isobutanol in the step (i) is 95%.

9. The method for producing methacrolein and/or methacrylic acid according to any one of claims 1 to 8, wherein oxidation reaction of isobutylene is performed at 200 to 400°C in the step (ii).

10. A method for producing methacrylic acid, comprising oxidizing methacrolein produced by the method according to any one of claims 1 to 9, to produce methacrylic acid.

11. A method for producing methacrylic acid ester, comprising esterifying methacrylic acid produced by the method according to any one of claims 1 to 10, to produce methacrylic acid ester.

## INTERNATIONAL SEARCH REPORT

| International application No. |
|---|
| **PCT/JP2023/005198** |

**A. CLASSIFICATION OF SUBJECT MATTER**

*C07C 45/35*(2006.01)i; *C07C 47/22*(2006.01)i; *C07C 51/215*(2006.01)i; *C07C 57/05*(2006.01)i; *C07B 61/00*(2006.01)i
FI:  C07C45/35; C07C47/22 A; C07C51/215; C07C57/05; C07B61/00 300

According to International Patent Classification (IPC) or to both national classification and IPC

**B. FIELDS SEARCHED**

Minimum documentation searched (classification system followed by classification symbols)

C07C45/35; C07C47/22; C07C51/215; C07C57/05; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2013-121946 A (MITSUBISHI RAYON CO., LTD.) 20 June 2013 (2013-06-20) claims, examples 1, 2, table 1, paragraphs [0033], [0036] | 1-11 |
| Y | WO 2013/069630 A1 (MITSUBISHI RAYON CO., LTD.) 16 May 2013 (2013-05-16) claims, example 1, comparative example 1, paragraphs [0007]-[0009], [0026], [0027] | 1-11 |
| Y | JP 50-13308 A (MITSUBISHI RAYON CO., LTD.) 12 February 1975 (1975-02-12) claims, examples 5, 6, comparative examples 3, 4 | 1-11 |
| Y | JP 48-32814 A (SUMITOMO CHEMICAL CO., LTD.) 02 May 1973 (1973-05-02) claims, comparative example 2 | 1-11 |
| Y | JP 58-189130 A (THE HALCON SD GROUP INC .) 04 November 1983 (1983-11-04) claims, p. 2, upper right, line 7 to last line, examples 1, 6, 8, fig. 2, 3 | 1-11 |
| Y | WO 2016/002649 A1 (MITSUBISHI RAYON CO., LTD.) 07 January 2016 (2016-01-07) claims, examples 1-20 | 1-11 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 March 2023** | **04 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2023/005198** |

**C.    DOCUMENTS CONSIDERED TO BE RELEVANT**

| Category* | Citation of document, with indication, where appropriate, of the relevant passages | Relevant to claim No. |
|---|---|---|
| A | TAYLOR, J. D. et al. Dehydration of Fermented Isobutanol for the Production of Renewable Chemicals and Fuels. Top Catal, 2010, vol. 53, pp. 1224-1230, DOI 10.1007/s11244-010-9567-8<br>    entire text, all drawings | 1-11 |
| A | JP 2021-192909 A (NIPPON KAYAKU KABUSHIKI KAISHA) 23 December 2021 (2021-12-23)<br>    entire text, all drawings | 1-11 |
| A | JP 2014-19675 A (NIPPON KAYAKU KABUSHIKI KAISHA) 03 February 2014 (2014-02-03)<br>    entire text, all drawings | 1-11 |

Form PCT/ISA/210 (second sheet) (January 2015)

<table>
<tr><td colspan="5" align="center">**INTERNATIONAL SEARCH REPORT**<br>Information on patent family members</td><td>International application No.<br><br>**PCT/JP2023/005198**</td></tr>
</table>

| Patent document<br>cited in search report | | | Publication date<br>(day/month/year) | Patent family member(s) | Publication date<br>(day/month/year) |
|---|---|---|---|---|---|
| JP | 2013-121946 | A | 20 June 2013 | (Family: none) | |
| WO | 2013/069630 | A1 | 16 May 2013 | US 2014/0357890 A1<br>claims, example 1,<br>comparative example 1,<br>paragraphs [0002]-[0004],<br>[0036]-[0039]<br>CN 103917508 A<br>KR 10-2014-0090650 A | |
| JP | 50-13308 | A | 12 February 1975 | US 3972920 A<br>claims, examples 5, 6,<br>comparative examples 3, 4 | |
| JP | 48-32814 | A | 02 May 1973 | (Family: none) | |
| JP | 58-189130 | A | 04 November 1983 | US 4413147 A<br>claims, 1st column, lines 6-19,<br>examples 1, 6, 8, fig. 2, 3 | |
| WO | 2016/002649 | A1 | 07 January 2016 | US 2017/0129844 A1<br>claims, examples 1-20<br>KR 10-2016-0148677 A | |
| JP | 2021-192909 | A | 23 December 2021 | CN 113939364 A<br>entire text, all drawings<br>KR 10-2022-0119386 A | |
| JP | 2014-19675 | A | 03 February 2014 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S4832814 A
- JP S5013308 A
- JP 2013121946 A
- WO 2013069630 A